# EUROPEAN PATENT APPLICATION

(11) **EP 4 257 557 A1**
(43) Date of publication of application: **11.10.2023**
(21) Application number: 22837237.1
(22) Date of filing: 08.03.2022
(51) Int. Cl.: C02F 1/44, C02F 1/461, C02F 1/68, G01N 27/416

(54) **METHOD FOR MEASURING DISSOLVED GAS CONCENTRATION, APPARATUS FOR MEASURING DISSOLVED HYDROGEN CONCENTRATION, AND APPARATUS FOR PRODUCING WATER FOR PREPARATION OF DIALYSATE**

(30) Priority: 06.07.2021 JP 2021112355
(71) Applicant: Nihon Trim Co., Ltd., Osaka 531-0076 (JP)
(72) Inventor: TACHIBANA Takahito, Nankoku-shi, Kochi 783-0060 (JP); KOIZUMI Yoshinobu, Nankoku-shi, Kochi 783-0060 (JP); MIYAKE Masato, Osaka-shi, Osaka 531-0076 (JP)
(74) Representative: Manitz Finsterwald Patent- und Rechtsanwaltspartnerschaft mbB
(86) International application number: PCT/JP2022/009895
(87) International publication number: WO 2023/281815

(57) **Abstract**

This apparatus 1 for measuring a dissolved hydrogen concentration is used for the purpose of measuring the dissolved hydrogen concentration in a hydrogen dissolved water into which a hydrogen gas has been dissolved. This apparatus 1 for measuring a dissolved hydrogen concentration comprises: a power generation cell 2 that comprises a first electrode chamber 21 into which a hydrogen dissolved water is supplied and a second electrode chamber 22 into which an oxygen gas is supplied, so as to generate electrical energy by having the hydrogen gas within the first electrode chamber 21 and the oxygen gas within the second electrode chamber 22 react with each other; a flow rate sensor 3 which senses the supply amount of the hydrogen dissolved water into the first electrode chamber 21; an electrical energy detection unit 4 which detects the electrical energy generated by the power generation cell 2; and a calculation unit 5 which calculates the dissolved hydrogen concentration on the basis of the supply amount sensed by the flow rate sensor 3 and the electrical energy detected by the electrical energy detection unit 4.

## Description

### Technical Field

The present invention relates to a method for measuring dissolved gas concentration, an apparatus for measuring dissolved hydrogen concentration, and an apparatus for producing water for preparation of dialysate.

### Background Art

Conventionally, hemodialysis treatment with electrolyzed water is of interest as being effective in reducing the oxidative stress of patients. In recent years of hemodialysis, it is desirable to be able to accurately know the dissolved hydrogen concentration of electrolyzed water used in the treatment.

In Patent Literature 1, an apparatus for measuring the concentration of dissolved hydrogen in a liquid to be measured has been proposed.

### Citation List

### Patent Literature

Patent Literature 1: Japanese Unexamined Patent Application Publication 2015-081799

### Summary of Invention

### Technical Problem

However, in the apparatus disclosed in the above-mentioned Patent Literature 1, it is difficult to measure the accurate dissolved hydrogen concentration in applications where the supply of the liquid to be measured fluctuates, and further improvement has been required.

The main object of the present invention is to provide a dissolved hydrogen concentration measurement technique capable of measuring an accurate dissolved hydrogen concentration even when used in applications where the supply amount of a liquid to be measured fluctuates.

### Solution to Problem

In one aspect of the present invention, a dissolved gas concentration measuring method for measuring a dissolved first gas concentration of a liquid in which the first gas is dissolved, comprises a first step of supplying the liquid to a first pole chamber of a power generation cell having the first pole chamber and a second pole chamber, a second step of detecting a supply amount of the liquid supplied to the first pole chamber, a third step of supplying a second gas generating electrical energy by reacting with the first gas to the second pole chamber of the power generation cell, a fourth step of reacting the first gas in the first pole chamber with the second gas in the second pole chamber to generate the electrical energy, a fifth step of detecting the electrical energy generated in the fourth step, and a sixth step of calculating the dissolved first gas concentration based on the supply amount of the liquid detected in the second step and the electrical energy detected in the fourth step.

In the present invention according to the dissolved gas concentration measuring method, it is preferable that the fourth step comprises releasing the first gas from the liquid.

In another aspect of the present invention, a dissolved hydrogen concentration measuring apparatus for measuring a dissolved hydrogen concentration of dissolved hydrogen water in which hydrogen gas is dissolved, comprises a power generation cell having a first pole chamber to which the dissolved hydrogen water is supplied and a second pole chamber to which oxygen gas is supplied, and generating electrical energy by reacting the hydrogen gas in the first pole chamber with the oxygen gas in the second pole chamber, a flow rate sensor for detecting an amount of dissolved hydrogen water supplied to the first pole chamber, an electrical energy detector for detecting the electrical energy generated by the power generation cell, and a calculator for calculating the dissolved hydrogen concentration based on the supply amount detected by the flow rate sensor and the electrical energy detected by the electrical energy detector.

In the dissolved hydrogen concentration measuring apparatus according to the present inventions, it preferably comprises a release device for releasing the hydrogen gas from the dissolved hydrogen water.

In the dissolved hydrogen concentration measuring apparatus according to the present invention, it preferably comprises an amplifier for amplifying the electrical energy generated by the power generation cell and outputting it as an electrical signal to the electrical energy detector.

In the dissolved hydrogen concentration measuring apparatus according to the present invention, it preferably comprises a noise removal device that removes noise components from the electrical signal amplified by the amplifier.

In another aspect of the present invention, a dialysate preparation water producing apparatus for producing dialysate preparation water, comprises a hydrogen dissolving device for dissolving hydrogen gas in raw water to generate dissolved hydrogen water, a reverse osmosis treatment device for producing dialysate preparation water for hemodialysis by reverse osmosis treatment of the dissolved hydrogen water, and the dissolved hydrogen concentration measuring apparatus as set forth in any one of claims 3 to 6 for measuring the dissolved hydrogen concentration of the dialysate preparation water produced by the reverse osmosis treatment device.

In the dialysate preparation water producing apparatus according to the present invention, it is preferable that the hydrogen dissolving device comprises an electrolytic cell for generating hydrogen gas by electrolyzing the raw water, and a first controller for controlling electrolytic current supplied to the electrolytic cell.

In the dialysate preparation water producing apparatus according to the present invention, it is preferable that the first controller controls the electrolytic current based on the dissolved hydrogen concentration calculated by the calculator.

In the dialysate preparation water producing apparatus according to the present invention, it is preferable that the hydrogen dissolving device comprises a hydrogen supply device for supplying a foamy hydrogen gas to the raw water, and a second controller for controlling the supply amount of the hydrogen gas.

In the dialysate preparation water producing apparatus according to the present invention, it is preferable that the second controller controls the supply amount of the hydrogen gas based on the dissolved hydrogen concentration calculated by the calculator.

In the dialysate preparation water producing apparatus according to the present invention, it preferably comprises a first water channel extending from the reverse osmosis treatment device to an intake for taking out the dialysate preparation water, and a second water channel branched from the first water channel and reaching a drain port via the power generation cell.

In the dialysate preparation water producing apparatus according to the present invention, it further comprises a bypass valve that controls the water flow from the first water channel to the second water channel.

### Advantageous Effects of Invention

In the dissolved gas concentration measuring method according to the present invention, the dissolved first gas concentration is calculated in the sixth step based on the supply amount of the liquid detected in the second step and the electrical energy detected in the fourth step. Therefore, even when used in applications where the supply amount of the liquid fluctuates, the dissolved first gas concentration can be accurately measured.

In the dissolved hydrogen concentration measuring apparatus according to the present invention, the calculator calculates the dissolved hydrogen concentration based on the supply amount detected by the flow rate sensor and the electrical energy detected by the electrical energy detector. Therefore, even when used in applications where the supply amount of the dissolved hydrogen water fluctuates, the dissolved hydrogen concentration can be accurately measured.

The dialysate preparation water producing apparatus according to the present invention comprises the hydrogen dissolving device for producing the dissolved hydrogen water, the reverse osmosis treatment device for reverse osmosis treatment of the dissolved hydrogen water, and a dissolved hydrogen concentration measuring apparatus for measuring the dissolved hydrogen concentration of the dialysate preparation water produced by the reverse osmosis treatment device. Therefore, the dissolved hydrogen concentration can be accurately measured even when used in applications where the production amount of the dialysate preparation water fluctuates.

### Brief Description of Drawings

FIG. 1 is a diagram showing a schematic configuration of a dissolved hydrogen concentration measuring apparatus according to an embodiment of the present invention.
FIG. 2 is a flow chart showing the procedure of a dissolved hydrogen concentration measuring method according to an embodiment of the present invention.
FIG. 3 is a diagram showing a schematic configuration of a modification of the dissolved hydrogen concentration measuring apparatus of FIG. 1.
FIG. 4 is a diagram showing a schematic configuration of a dialysate preparation water producing apparatus including the dissolved hydrogen concentration measuring apparatus of FIG. 3.
FIG. 5 is a diagram showing a schematic configuration of one embodiment of the hydrogen dissolving device of FIG. 4.
FIG. 6 is a diagram showing a schematic configuration of a modification of the hydrogen dissolving device of FIG. 5.

### Description of Embodiments

An embodiment of the present invention will be described below with reference to the drawings.

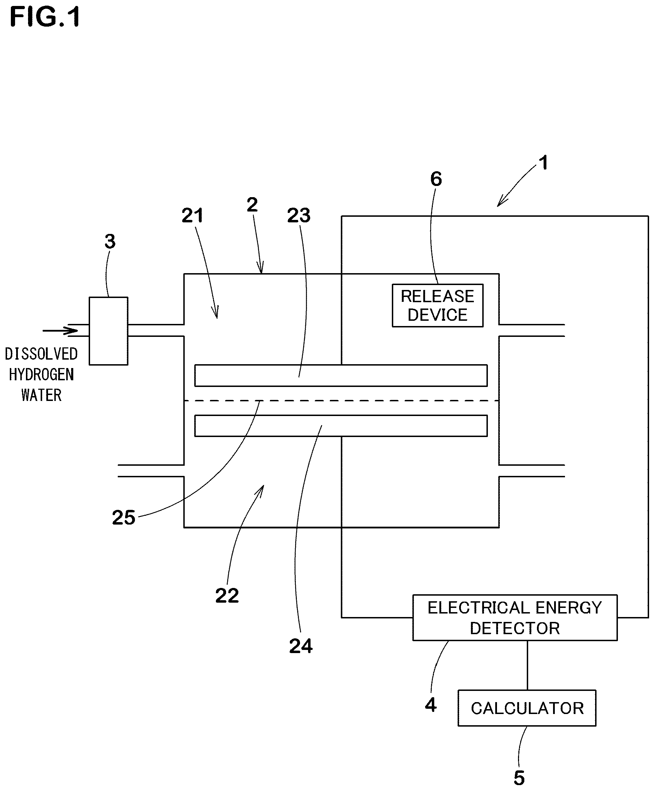

FIG. 1 shows a configuration of a dissolved hydrogen concentration measuring apparatus 1 according to the present embodiment. The dissolved hydrogen concentration measuring apparatus 1 is an apparatus for measuring a dissolved hydrogen concentration of dissolved hydrogen water in which hydrogen gas is dissolved.

The dissolved hydrogen concentration measuring apparatus 1 includes a power generation cell 2, a flow rate sensor 3, an electrical energy detector 4, and a calculator 5.

The power generation cell 2 includes a first pole chamber 21 to which the dissolved hydrogen water is supplied and a second pole chamber 22 to which oxygen gas is supplied. A first electrode 23 is disposed in the first pole chamber 21 and a second electrode 24 is disposed in the second pole chamber 22. An electrolyte membrane 25 made of a solid polymer film or the like is disposed between the first electrode 23 and the second electrode 24. The electrolyte membrane 25 divides the inside of the power generation cell 2 into a first pole chamber 21 and a second pole chamber 22.

The power generation cell 2 generates electrical energy (electric power) by reacting hydrogen gas in the first pole chamber 21 with oxygen gas in the second pole chamber 22. With respect to the operation of the power generation cell 2, it is similar to the power generation principle of the fuel cell. The electrical energy generated in the power generation cell 2 may be extracted from the first electrode 23 and the second electrode 24.

For example, the flow rate sensor 3 is provided in a flow path for supplying dissolved hydrogen water to the first pole chamber 21. The flow rate sensor 3 detects the amount of dissolved hydrogen water supplied to the first pole chamber 21 and outputs an electric signal corresponding to the supply amount to the calculator 5.

The electrical energy detector 4 is connected to the first electrode 23 and the second electrode 24 of the power generation cell 2. The electrical energy detector 4 detects the electrical energy generated by the power generation cell 2 based on the voltage and current between the first electrode 23 and the second electrode 24, and the electrical energy detector 4 outputs an electric signal corresponding to the electrical energy to the calculator 5.

The calculator 5 is connected to the flow rate sensor 3 and the electrical energy detector 4. For example, a microcomputer chip having a processor and a memory is applied to the calculator 5.

The calculator 5 calculates the dissolved hydrogen concentration based on the supply amount detected by the flow rate sensor 3 and the electrical energy detected by the electrical energy detector 4. The dissolved hydrogen concentration calculated by the calculator 5 is output to a display unit (not shown) such as a liquid crystal panel, for example. A storage unit for storing the dissolved hydrogen concentration calculated by the calculator 5 may be provided.

The electrical energy generated by the power generation cell 2 depends on the filling amount of the hydrogen gas into the first pole chamber 21, that is, the dissolved hydrogen concentration and supply amount of the dissolved hydrogen water supplied to the first pole chamber 21, and the filling amount of the oxygen gas into the second pole chamber 22, that is, the supply amount of oxygen gas to the second pole chamber 22. Therefore, when the supply amount of the oxygen gas is made constant, the electrical energy has a fixed correlation between the dissolved hydrogen concentration and the supply amount of the dissolved hydrogen water supplied to the first pole chamber 21, and reproducibility is recognized therein.

The inventors of the present application have found that the dissolved hydrogen concentration of dissolved hydrogen water can be calculated by measuring the flow rate of dissolved hydrogen water flowing into the first pole chamber 21 and the electrical energy generated by the power generation cell 2 while keeping the concentration of oxygen gas in the second pole chamber 22 constant as a result of intensive studies. In calculating the dissolved hydrogen concentration, it is necessary to specify the relationship among the electrical energy, the dissolved hydrogen concentration, and the supply amount of dissolved hydrogen water in advance. The relationship among the electrical energy, the dissolved hydrogen concentration, and the supply amount of dissolved hydrogen water can be specified from the supply amount and the electrical energy detected by the flow rate sensor 3 and the electrical energy detector 4, respectively, by supplying dissolved hydrogen water having a known dissolved hydrogen concentration to the first pole chamber 21.

In the dissolved hydrogen concentration measuring apparatus 1, the calculator 5 calculates the dissolved hydrogen concentration based on the supply amount of dissolved hydrogen water detected by the flow rate sensor 3 and the electrical energy detected by the electrical energy detector 4. Therefore, even when used in applications where the supply amount of dissolved hydrogen water fluctuates, the dissolved hydrogen concentration can be accurately measured.

In the dissolved hydrogen concentration measuring apparatus 1, it is preferable to include a release device 6 for releasing the hydrogen gas from the dissolved hydrogen water. The release of hydrogen gas from the dissolved hydrogen water promotes the reaction with oxygen gas, and which is detected as electrical energy by the electrical energy detector 4. Accordingly, the concentration of hydrogen gas dissolved in dissolved hydrogen water is accurately measured.

The release device 6 according to the present embodiment includes a heater for heating the dissolved hydrogen water. The heater heats the dissolved hydrogen water in, or supplied to, the first pole chamber 21. By heating the dissolved hydrogen water by the heater, the collision of the molecules constituting the dissolved hydrogen water becomes violent and the hydrogen gas is released from the dissolved hydrogen water. The release device 6 may include a configuration for agitating the dissolved hydrogen water instead of or in addition to the heater.

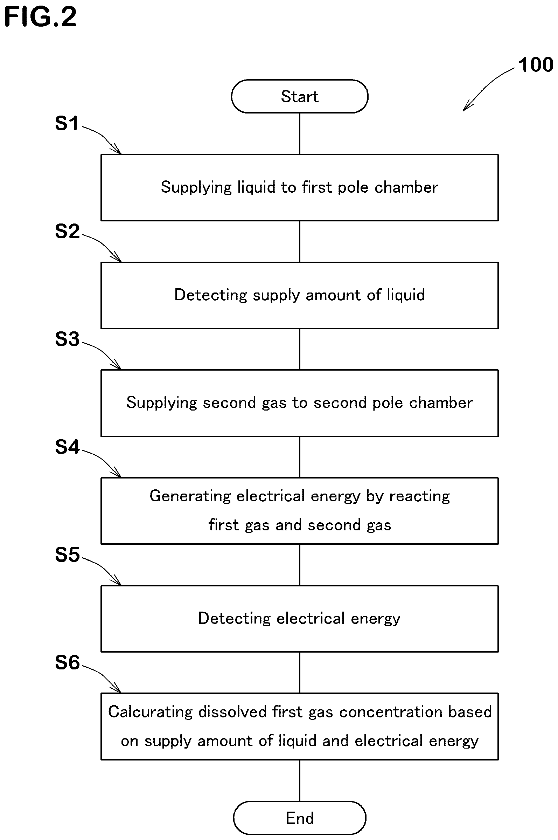

FIG. 2 shows the procedure of a dissolved gas concentration measuring method 100 using the dissolved hydrogen concentration measuring apparatus 1. The dissolved gas concentration measuring method 100 is a method for measuring a dissolved first gas concentration of a liquid in which the first gas is dissolved. In the present embodiment, hydrogen gas is applied as the first gas. The first gas is not limited to hydrogen gas as long as it is a gas that reacts with the second gas to generate electrical energy. Further, although water is applied as the liquid above, the liquid is not limited to water as long as it is the liquid in which the first gas is dissolved.

The dissolved gas concentration measuring method 100 includes the first step S1 to the sixth step S6.

In the first step S 1, a liquid is supplied to the first pole chamber 21 of a power generation cell 2 having the first pole chamber 21 and the second pole chamber 22. In the present embodiment, dissolved hydrogen water is supplied to the first pole chamber 21 of the power generation cell 2.

In the second step S2, the supply amount of the liquid supplied to the first pole chamber 21 is detected. In this embodiment, the flow rate sensor 3 detects the supply amount of dissolved hydrogen water.

In the third step S3, the second gas is supplied to the second pole chamber 22 of the power generation cell 2. In the present embodiment, oxygen gas, which generates electrical energy by reacting with hydrogen gas in the first pole chamber, is supplied to the second pole chamber. However, it is not limited to oxygen gas as long as it is a gas that reacts with the first gas to generate electrical energy.

In the third step S3, since the second gas is sufficient to exist in the second pole chamber 22, the liquid containing the second gas may be supplied to the second pole chamber 22. For example, water (dissolved oxygen water) in which oxygen gas is dissolved may be supplied to the second pole chamber 22. Preferably, in the fourth step S4 below, a sufficient amount of the second gas is continuously supplied to the second pole chamber 22 so that the generation of electrical energy is not delayed due to the shortage of the second gas.

In the fourth step S4, the first gas in the first pole chamber 21 and the second gas in the second pole chamber 22 are reacted to generate electrical energy. In this embodiment, the hydrogen gas in the first pole chamber 21 reacts with the oxygen gas in the second pole chamber 22 to generate electrical energy.

In the fifth step S5, the electrical energy generated in the fourth step S4 is detected. In the present embodiment, an electrical energy detector 4 detects the electrical energy.

In the sixth step S6, the dissolved first gas concentration is calculated based on the supply amount of the liquid detected in the second step S2 and the electrical energy detected in the fifth step S5. In the present embodiment, the calculator 5 calculates the dissolved hydrogen concentration based on the supply amount of dissolved hydrogen water and the electrical energy detected by the electrical energy detector 4.

In the dissolved gas concentration measuring method 100, the dissolved first gas concentration is calculated in the sixth step S6 based on the supply amount of the liquid detected in the second step S2 and the electrical energy detected in the fifth step S5. Therefore, even when used in applications where the supply of liquid fluctuates, the dissolved first gas concentration can be accurately measured.

In the dissolved gas concentration measuring method 100, in a configuration in which the dissolved hydrogen concentration measuring apparatus 1 including the release device 6 is applied, the fourth step S4 may include the step of releasing the first gas from the liquid supplied in the first step S1. The step is achieved by heating the liquid.

In such a dissolved gas concentration measuring method 100, the first gas is released from the liquid in the fourth step S4, whereby the reaction with the second gas is promoted and detected as electrical energy in the fifth step S5. Accordingly, the concentration of the first gas dissolved in the liquid is accurately measured.

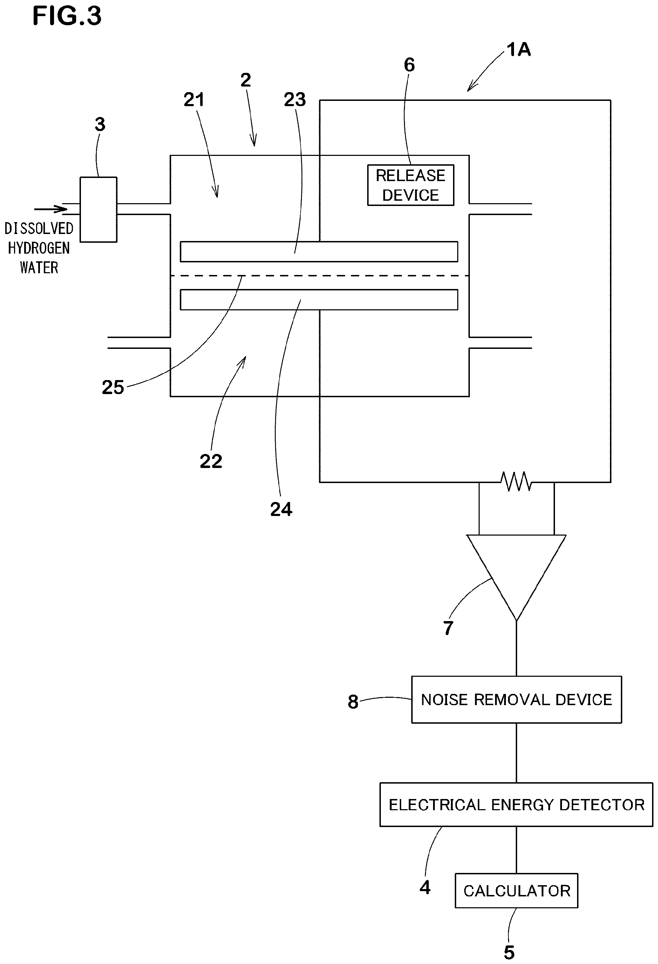

FIG. 3 is a diagram showing a schematic configuration of the dissolved hydrogen concentration measuring apparatus 1A, which is a modification of the dissolved hydrogen concentration measuring apparatus 1 of FIG. 1. The configurations of the dissolved hydrogen concentration measuring apparatus 1 described above may be employed for the portions of the dissolved hydrogen concentration measuring apparatus 1Anot described below.

The dissolved hydrogen concentration measuring apparatus 1A further includes an amplifier 7. The amplifier 7 includes an amplifier circuit. The amplifier 7 is arranged between the first electrode 23 and the second electrode 24 of the power generation cell 2 and the electrical energy detector 4.

The amplifier 7 amplifies the electrical energy generated by the power generation cell 2 and outputs it as an electrical signal to the electrical energy detector 4. This makes it possible to detect the minute electrical energy by the electrical energy detector 4, and increases the measurement accuracy of the dissolved hydrogen concentration.

Preferably, the dissolved hydrogen concentration measuring apparatus 1A further includes a noise removal device 8. The noise removal device 8 is arranged between the amplifier 7 and the electrical energy detector 4. A filter circuit, such as a low-pass filter, a bandpass filter or a high-pass filter, is applied to the noise removal device 8.

The noise removal device 8 removes noise components of a particular frequency from the electrical signals amplified by the amplifier 7. This improves the detection accuracy of the electric energy by the electrical energy detector 4.

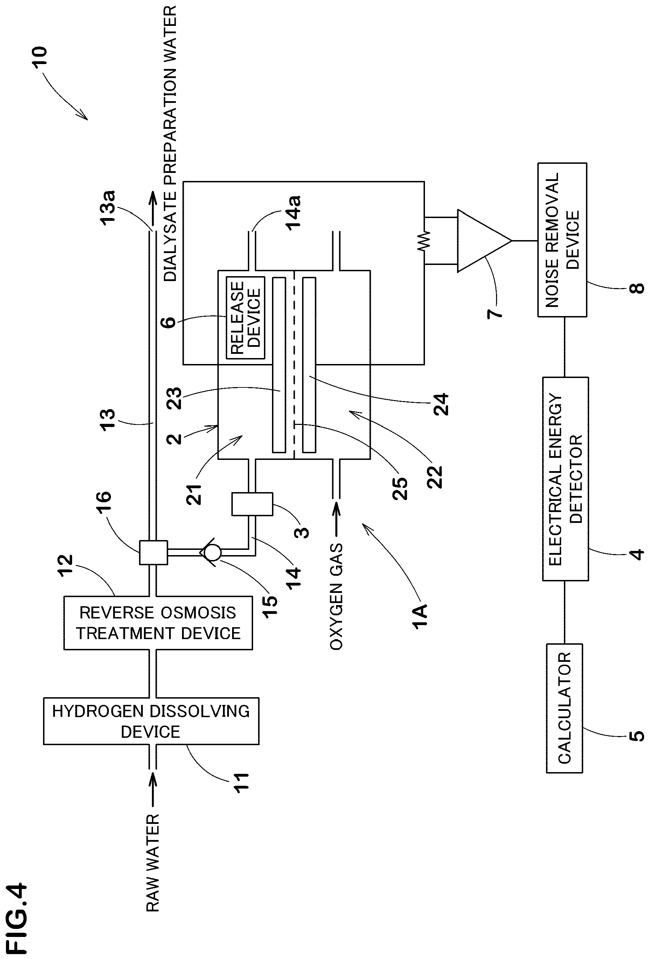

FIG. 4 shows a diagram of a schematic configuration of a dialysate preparation water producing apparatus 10 including the dissolved hydrogen concentration measuring apparatus 1A. The dialysate preparation water producing apparatus 10 is an apparatus for producing dialysate preparation water using dissolved hydrogen water. The dissolved hydrogen concentration measuring apparatus 1 may be applied instead of the dissolved hydrogen concentration measuring apparatus 1A.

The dialysate preparation water producing apparatus 10 includes a hydrogen dissolving device 11 and a reverse osmosis treatment device 12.

The hydrogen dissolving device 11 generates dissolved hydrogen water by dissolving hydrogen gas in raw water. The raw water is supplied from the outside of the dialysate preparation water producing apparatus 10. Tap water, well water or ground water may be used for the raw water. It is preferable that the raw water supplied to the hydrogen dissolving device 11 is purified by a filter or the like, and the hypochlorous acid is removed by activated carbon or the like.

The dissolved hydrogen water produced by the hydrogen dissolving device 11 is supplied to the reverse osmosis treatment device 12. The reverse osmosis treatment device 12 has a reverse osmosis membrane. The reverse osmosis treatment device 12 produces dialysate preparation water for hemodialysis by reverse osmosis treatment of dissolved hydrogen water, that is, pressurizing dissolved hydrogen water to pass through the reverse osmosis membrane. A dialysate is produced by using the reverse osmosis treated water.

The dissolved hydrogen concentration measuring apparatus 1A measures the dissolved hydrogen concentration of the dialysate preparation water produced by the reverse osmosis treatment device 12. Therefore, the dissolved hydrogen concentration can be accurately measured even when the dialysate preparation water producing apparatus 10 is used in applications where the producing amount of the dialysate preparation water fluctuates.

The dialysate preparation water producing apparatus 10 includes a first water channel 13 and a second water channel 14.

The first water channel 13 extends from the reverse osmosis treatment device 12 to an intake 13a for taking out the dialysate preparation water. The intake 13a is connected to a dialysate generator (not shown) external to the dialysate preparation water producing apparatus 10. The dialysate generator produces dialysate solution by mixing (or dissolving dialysate component powders) the dialysate stock solution in the dialysate preparation water.

The second water channel 14 is branched from the first water channel 13 and reaches a drain port 14a via the power generation cell 2 of the dissolved hydrogen concentration measuring apparatus 1A. That is, the dialysate preparation water produced by the reverse osmosis treatment device is supplied to the power generation cell 2 via the second water channel 14. This enables to measure the dissolved hydrogen concentration in the dialysate preparation water by the dissolved hydrogen concentration measuring apparatus 1A.

It is preferable that a check valve 15 is provided in front of the first pole chamber 21 in the first water channel 13. It is also preferable to exhaust the dialysate preparation water discharged from the drain port 14a via the first pole chamber 21 without merging with the dialysate preparation water in the first water channel 13. As a result, the dialysate preparation water supplied from the first water channel 13 to the dialysate generator via the intake 13a is independent of the dialysate preparation water that has flowed into the power generation cell 2 from the second water channel 14, and its cleanliness is easily ensured.

It is preferable that a bypass valve 16 for controlling the water flow from the first water channel 13 to the second water channel 14 is provided in the branching part to the second water channel 14. A solenoid valve or the like is applied to the bypass valve 16. The bypass valve 16 is controlled, for example, by a calculator 5

By closing the bypass valve 16, the water flow from the first water channel 13 to the second water channel 14 is blocked. All of the dialysate preparation water produced in the reverse osmosis treatment device 12 is supplied to the dialysate generator via the intake 13a. On the other hand, by opening the bypass valve 16, a part of the dialysate preparation water generated by the reverse osmosis treatment device 12 flows into the second water channel 14, and the dissolved hydrogen concentration is measured by the dissolved hydrogen concentration measuring apparatus 1A. Therefore, by limiting the opening of the bypass valve 16 when measuring the concentration of dissolved hydrogen, it is possible to reduce the dialysate preparation water discharged from the drain port 14a and to effectively utilize the dialysate preparation water.

After being subjected to the measurement of the dissolved hydrogen concentration in the power generation cell 2, the water discharged from the drain port 14a may be returned to the hydrogen dissolving device 11.

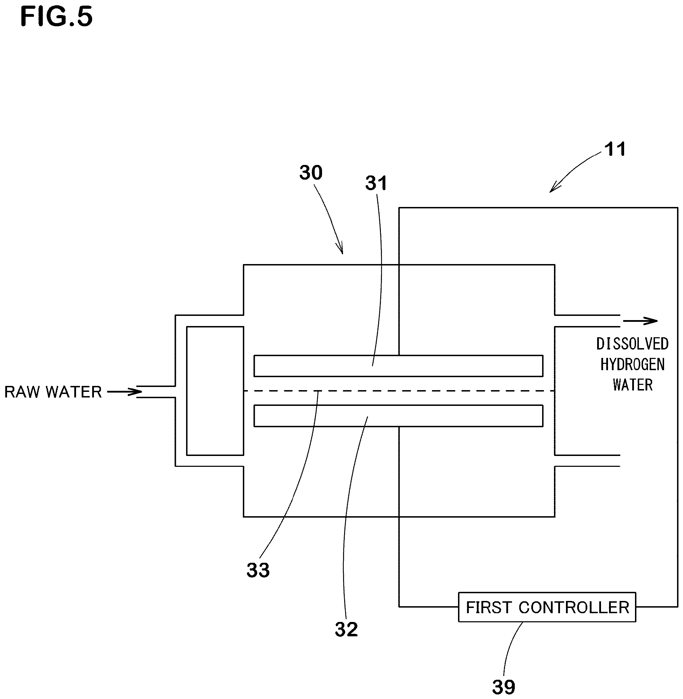

FIG. 5 shows an embodiment of the hydrogen dissolving device 11. The hydrogen dissolving device 11 includes an electrolytic cell 30 and a first controller 39.

The electrolytic cell 30 generates hydrogen gas by electrolyzing the supplied raw water. The electrolytic cell 30 has a first power feeder 31 and a second power feeder 32 in an electrolytic chamber. The polarity of the first power feeder 31 and the second power feeder 32 and the voltage applied to the first power feeder 31 and the second power feeder 32 are controlled by the first controller 39.

A diaphragm 33 is provided between the first power feeder 31 and the second power feeder 32. For example, a solid polymer membrane comprising a fluorine-based resin having a sulfonic acid group is used as appropriate for the diaphragm 33. The solid polymer membrane, by electrolysis, moves the oxonium ions generated on the anode side to the cathode side to produce a hydrogen gas product. Therefore, the pH of the dissolved hydrogen water does not change without generating hydroxide ions during electrolysis.

Dissolved hydrogen water is generated in the electrolytic cell 30 by dissolving hydrogen gas generated by electrolysis into water on the cathode side.

The first controller 39 controls the electrolytic current supplied to the electrolytic cell 30 (i.e., the first power feeder 31 and the second power feeder 32) by controlling the DC voltage applied to the first power feeder 31 and the second power feeder 32. More specifically, the first controller 39 feedback-controls the DC voltage applied to the first power feeder 31 and the second power feeder 32 so that the electrolytic current becomes a predetermined desired value. For example, if the electrolytic current is excessive, the first controller 39 decreases the voltage and if the electrolytic current is too small, the first controller 39 increases the voltage. This appropriately controls the electrolytic current supplied to the first power feeder 31 and the second power feeder 32.

The first controller 39 and the calculator 5 are connected to each other. Preferably, the first controller 39 is configured to control the electrolytic current based on the dissolved hydrogen concentration calculated by the calculator 5.

For example, if the dissolved hydrogen concentration calculated by the calculator 5 is insufficient for a predetermined target value, the first controller 39 increases the electrolytic current supplied to the first power feeder 31 and the second power feeder 32. On the other hand, if the dissolved hydrogen concentration calculated by the calculator 5 is excessive with respect to the target value, the first controller 39 reduces the electrolytic current supplied to the first power feeder 31 and the second power feeder 32. This stabilizes the dissolved hydrogen concentration of the dialysate preparation water and easily produces dialysate preparation water with a dissolved hydrogen concentration optimal for treatment.

For example, a microcomputer chip similar to the calculator 5 is applied to the first controller 39. If there is a margin in the processing capacity of the calculator 5, the functions of the first controller 39 may be integrated into the calculator 5. This simplifies the configuration of the dialysate preparation water producing apparatus 10.

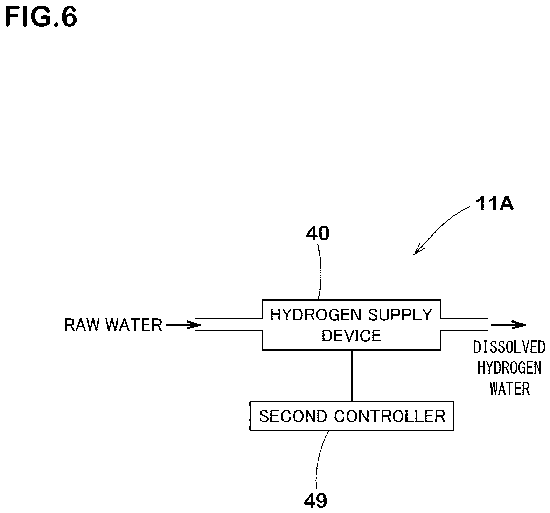

FIG. 6 shows a hydrogen dissolving device 11A which is a modification of the hydrogen dissolving device 11. The hydrogen dissolving device 11A includes a hydrogen supply device 40 and a second controller 49.

The hydrogen supply device 40 supplies a foamy hydrogen gas to the supplied raw water. For example, the hydrogen supply device 40 is configured to include a cylinder filled with hydrogen gas. Dissolved hydrogen water in which hydrogen gas is dissolved is generated by hydrogen supply device 40.

The second controller 49 controls the amount of hydrogen gas supplied by the hydrogen supply device 40. The dissolved hydrogen concentration is increased by the second controller 49 by increasing the supply amount of the hydrogen gas supplied from the hydrogen supply device 40. On the other hand, the second controller 49 decreases the dissolved hydrogen concentration by reducing the supply amount of the hydrogen gas supplied from the hydrogen supply device 40.

The second controller 49 and the calculator 5 are connected to each other. Preferably, the second controller 49 is configured to control the electrolytic current based on the dissolved hydrogen concentration calculated by the calculator 5.

For example, if the dissolved hydrogen concentration calculated by the calculator 5 is insufficient for a predetermined target value, the second controller 49 increases the supply amount of the hydrogen gas supplied from the hydrogen supply device 40. On the other hand, if the dissolved hydrogen concentration calculated by the calculator 5 is excessive with respect to the target value, the second controller 49 reduces the supply amount of the hydrogen gas supplied from the hydrogen supply device 40. This stabilizes the dissolved hydrogen concentration of the dialysate preparation water and easily produces dialysate preparation water with a dissolved hydrogen concentration optimal for treatment.

For example, a microcomputer chip similar to the calculator 5 is applied to the second controller 49. If there is a margin in the processing capacity of the calculator 5, the function of the second controller 49 may be integrated into the calculator 5. This simplifies the configuration of the dialysate preparation water producing apparatus 10.

Although the dissolved hydrogen concentration measuring apparatus and other apparatus have been described in detail above, the present invention is not limited to the specific embodiments described above, but may be modified and implemented in various ways.

That is, the dissolved gas concentration measuring method 100 is a method for measuring a dissolved first gas concentration of a liquid in which the first gas is dissolved, the method, at least, may include a first step S1 of supplying the liquid to a first pole chamber 21 of a power generation cell 2 having the first pole chamber 21 and a second pole chamber 22; a second step S2 of detecting a supply amount of the liquid supplied to the first pole chamber 21; a third step S3 of supplying a second gas generating electrical energy by reacting with the first gas to the second pole chamber 22 of the power generation cell 2; a fourth step S4 of reacting the first gas in the first pole chamber 21 with the second gas in the second pole chamber 22 to generate the electrical energy; a fifth step S5 of detecting the electrical energy generated in the fourth step S4; and a sixth step S6 of calculating the dissolved first gas concentration based on the supply amount of the liquid detected in the second step S2 and the electrical energy detected in the fourth step S4.

In addition, the dissolved hydrogen concentration measuring apparatus 1 is an apparatus for measuring a dissolved hydrogen concentration of dissolved hydrogen water in which hydrogen gas is dissolved, the apparatus, at least, may include a power generation cell 2 having a first pole chamber 21 to which the dissolved hydrogen water is supplied and a second pole chamber 22 to which oxygen gas is supplied, and generating electrical energy by reacting the hydrogen gas in the first pole chamber 21 with the oxygen gas in the second pole chamber 22; a flow rate sensor 3 for detecting an amount of dissolved hydrogen water supplied to the first pole chamber 21; an electrical energy detector 4 for detecting the electrical energy generated by the power generation cell 2; and a calculator 5 for calculating the dissolved hydrogen concentration based on the supply amount detected by the flow rate sensor 3 and the electrical energy detected by the electrical energy detector 4.

Furthermore, the dialysate preparation water producing apparatus 10 is an apparatus for producing dialysate preparation water, the apparatus, at least, may include a hydrogen dissolving device 11 for dissolving hydrogen gas in raw water to generate dissolved hydrogen water; a reverse osmosis treatment device 12 for producing dialysate preparation water for hemodialysis by reverse osmosis treatment of the dissolved hydrogen water; and a dissolved hydrogen concentration measuring apparatus 1 for measuring the dissolved hydrogen concentration of the dialysate preparation water produced by the reverse osmosis treatment device 12.

### Reference Signs List

- 1: dissolved hydrogen concentration measuring apparatus
- 1A: dissolved hydrogen concentration measuring apparatus
- 2: power generation cell
- 3: flow rate sensor
- 4: electrical energy detector
- 5: calculator
- 6: release device
- 7: amplifier
- 8: noise removal device
- 10: dialysate preparation water producing apparatus
- 11: hydrogen dissolving device
- 11A: hydrogen dissolving device
- 12: reverse osmosis treatment device
- 13: first water channel
- 13a: intake
- 14: second water channel
- 14a: drain port
- 16: bypass valve
- 21: first pole chamber
- 22: second pole chamber
- 30: electrolytic cell
- 39: first controller
- 40: hydrogen supply device
- 49: second controller
- 100: dissolved gas concentration measuring method
- S1: first step
- S2: second step
- S3: third step
- S4: fourth step
- S5: fifth step
- S6: sixth step

## Claims

1. A dissolved gas concentration measuring method for measuring a dissolved first gas concentration of a liquid in which the first gas is dissolved, comprising:
a first step of supplying the liquid to a first pole chamber of a power generation cell having the first pole chamber and a second pole chamber;
a second step of detecting a supply amount of the liquid supplied to the first pole chamber;
a third step of supplying a second gas generating electrical energy by reacting with the first gas to the second pole chamber of the power generation cell;
a fourth step of reacting the first gas in the first pole chamber with the second gas in the second pole chamber to generate the electrical energy;
a fifth step of detecting the electrical energy generated in the fourth step; and
a sixth step of calculating the dissolved first gas concentration based on the supply amount of the liquid detected in the second step and the electrical energy detected in the fourth step.

2. The dissolved gas concentration measuring method as set forth in claim 1, wherein
the fourth step comprises releasing the first gas from the liquid.

3. A dissolved hydrogen concentration measuring apparatus for measuring a dissolved hydrogen concentration of dissolved hydrogen water in which hydrogen gas is dissolved, comprising:
a power generation cell having a first pole chamber to which the dissolved hydrogen water is supplied and a second pole chamber to which oxygen gas is supplied, and generating electrical energy by reacting the hydrogen gas in the first pole chamber with the oxygen gas in the second pole chamber;
a flow rate sensor for detecting an amount of dissolved hydrogen water supplied to the first pole chamber;
an electrical energy detector for detecting the electrical energy generated by the power generation cell; and
a calculator for calculating the dissolved hydrogen concentration based on the supply amount detected by the flow rate sensor and the electrical energy detected by the electrical energy detector.

4. The dissolved hydrogen concentration measuring apparatus as set forth in claim 3, comprising a release device for releasing the hydrogen gas from the dissolved hydrogen water.

5. The dissolved hydrogen concentration measuring apparatus as set forth in claim 3 or 4, further comprising an amplifier for amplifying the electrical energy generated by the power generation cell and outputting it as an electrical signal to the electrical energy detector.

6. The dissolved hydrogen concentration measuring apparatus as set forth in claim 5, further comprising a noise removal device that removes noise components from the electrical signal amplified by the amplifier.

7. A dialysate preparation water producing apparatus for producing dialysate preparation water, comprising:
a hydrogen dissolving device for dissolving hydrogen gas in raw water to generate dissolved hydrogen water;
a reverse osmosis treatment device for producing dialysate preparation water for hemodialysis by reverse osmosis treatment of the dissolved hydrogen water; and
the dissolved hydrogen concentration measuring apparatus as set forth in any one of claims 3 to 6 for measuring the dissolved hydrogen concentration of the dialysate preparation water produced by the reverse osmosis treatment device.

8. The dialysate preparation water producing apparatus as set forth in claim 7, wherein the hydrogen dissolving device comprises
an electrolytic cell for generating hydrogen gas by electrolyzing the raw water, and
a first controller for controlling electrolytic current supplied to the electrolytic cell.

9. The dialysate preparation water producing apparatus as set forth in claim 8, wherein
the first controller controls the electrolytic current based on the dissolved hydrogen concentration calculated by the calculator.

10. The dialysate preparation water producing apparatus as set forth in claim 7, wherein
the hydrogen dissolving device comprises
a hydrogen supply device for supplying a foamy hydrogen gas to the raw water, and
a second controller for controlling the supply amount of the hydrogen gas.

11. The dialysate preparation water producing apparatus as set forth in claim 10, wherein
the second controller controls the supply amount of the hydrogen gas based on the dissolved hydrogen concentration calculated by the calculator.

12. The dialysate preparation water producing apparatus as set forth in any one of claims 7 to 11, comprising
a first water channel extending from the reverse osmosis treatment device to an intake for taking out the dialysate preparation water, and
a second water channel branched from the first water channel and reaching a drain port via the power generation cell.

13. The dialysate preparation water producing apparatus as set forth in claim 12, further comprising a bypass valve that controls the water flow from the first water channel to the second water channel.
